# EUROPEAN PATENT SPECIFICATION

(11) **EP 3 579 926 B1**
(45) Date of publication and mention of the grant of the patent: **09.12.2020**
(21) Application number: 18702313.0
(22) Date of filing: 05.02.2018
(51) Int. Cl.: A61Q 5/00, A61K 8/44

(54) **METHOD OF STRENGTHENING HAIR**
VERFAHREN ZUR STÄRKUNG VON HAAREN
PROCÉDÉ DE RENFORCEMENT DE CHEVEUX

(30) Priority: 13.02.2017 EP 17155882
(43) Date of publication of application: 18.12.2019
(73) Proprietor: Unilever PLC, London, Greater London EC4Y 0DY (GB); Unilever N.V., 3013 AL Rotterdam (NL)
(72) Inventor: PAUL, Prem, Kumar, Cheyalazhagan, Bebington Wirral Merseyside CH63 3JW (GB); ROGERS, Charlotte, Breony, Tandy, Bebington Wirral Merseyside CH63 3JW (GB)
(74) Representative: Chisem, Janet
(86) International application number: PCT/EP2018/052835
(87) International publication number: WO 2018/146054

(56) References cited:
- EP-A1- 0 422 765
- EP-A1- 1 493 423
- WO-A1-00/40217
- CA-A1- 2 023 090
- CA-A1- 2 144 912
- FR-A1- 2 912 650
- DATABASE WPI Week 201554 Thomson Scientific, London, GB; AN 2015-351385 XP002769643, & KR 2015 0062590 A (AMOREPACIFIC CORP) 8 June 2015 (2015-06-08)

## Description

### Field of the Invention

This invention relates to a method of strengthening the fibres of oxidatively treated hair.

### Background of the Invention

The hair fibre is susceptible to damage from a number of sources. These include environmental factors such as excessive exposure to UV; chemical factors such as bleaching or other oxidative processes; and mechanical factors such as overuse of heated styling appliances. Over time, the cumulative effect of these damaging factors may weaken the hair fibre. Weak hair tends to break easily, resulting in split ends and a ragged hairstyle. Weak hair may also appear limp, frizzy or flyaway, or be less capable of holding a style.

Film-forming polymers are often used in treatments for styling and conditioning hair because they alter hair surface properties, imparting smoothing and gliding effects and shine, and have a significant impact on the macroscopic behavior of the hair array. However, film-forming polymers are by nature designed to provide hair fibres with a hydrophobic coating that may slow or prevent the penetration of actives. Therefore, such treatments may not provide intrinsic benefits to the fibre such as strengthening.

WO00/40217 discloses aqueous compositions containing N-acetyl amino acids such as N-acetyl lysine used for alleviating or improving various cosmetic and dermatological conditions or disorders including changes and damages to skin, nail and hair associated with intrinsic and/or extrinsic ageing as well as changes or damages caused by extrinsic factors such as sunlight, radiation, air pollution, wind, cold, heat, dampness, chemicals, smoke and cigarette smoking. Exemplified compositions include an aqueous treatment composition comprising water, ethanol, propylene glycol and 0.5 wt.-% N-acetyl lysine.

The present invention addresses this problem.

### Summary of the Invention

The present invention provides a method of strengthening the fibres of oxidatively treated hair, the method comprising the sequential steps of:
(i) washing the hair;
(ii) soaking the washed hair in an aqueous treatment composition, and
(iii) drying the soaked hair;
characterised in that the aqueous treatment composition comprises at least 1% N-acetyl lysine (by weight based on the total weight of the composition).

The invention also provides the use of an aqueous treatment composition comprising at least 1% N-acetyl lysine (by weight based on the total weight of the composition), for the strengthening of oxidatively treated hair fibres.

### Detailed Description and Preferred Embodiments

All molecular weights as used herein are weight average molecular weights, unless otherwise specified.

The aqueous treatment composition for use in step (ii) of the method of the invention will typically comprise an aqueous continuous phase.

By "aqueous continuous phase" is meant a continuous phase which has water as its basis. Accordingly, the aqueous treatment composition will generally comprise at least 60%, preferably at least 70% and more preferably at least 80% water (by weight based on the total weight of the composition). The composition comprises no more than 99% and preferably no more than 98% water (by weight based on the total weight of the composition). Other organic solvents may also be present, such as lower alkyl alcohols and polyhydric alcohols. Examples of lower alkyl alcohols include C₁ to C₆ monohydric alcohols such as ethanol and isopropanol. Examples of polyhydric alcohols include propylene glycol, hexylene glycol, glycerin, and propanediol. Mixtures of any of the above described organic solvents may also be used.

The aqueous treatment composition for use in step (ii) of the method of the invention comprises at least 1% N-acetyl lysine (by weight based on the total weight of the composition).

N-acetyl lysine is an acetyl derivative of the amino acid lysine. N-acetyl lysine occurs in a number of isomeric forms. The preferred isomeric form of N-acetyl lysinel for use in step (ii) of the method of the invention is N-α-acetyl-L-lysine. N-α-acetyl-L-lysine has the following structural formula:

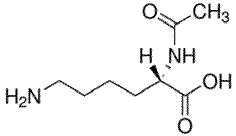

Preferably the N-acetyl lysine is used at a level ranging from 1 to 6%, more preferably from 1 to 3% and most preferably from 1.5 to 2.5% (by weight based on the total weight of the composition).

An especially preferred aqueous treatment composition for use in step (ii) of the method of the invention comprises N-α-acetyl-L-lysine at a level ranging from 1.5 to 2.5% (by weight based on the total weight of the composition).

An aqueous treatment composition for use in step (ii) of the method of the invention may suitably include a conditioning gel phase, which may be generally characterized as a gel (Lβ) surfactant mesophase consisting of surfactant bilayers. Such a conditioning gel phase may be formed from a cationic surfactant, a high melting point fatty alcohol and an aqueous carrier. Typically, these components are heated to form a mixture, which is cooled under shear to room temperature. The mixture undergoes a number of phase transitions during cooling, normally resulting in a gel (L_{β}) surfactant mesophase consisting of surfactant bilayers.

Examples of suitable cationic surfactants which are useful for forming the conditioning gel phase include quaternary ammonium cationic surfactants corresponding to the following general formula:

[N(R¹)(R²)(R³)(R⁴)]⁺ (X)⁻

in which R¹, R², R³, and R⁴ are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halide, (e.g. chloride, bromide), acetate, citrate, lactate, glycolate, phosphate nitrate, sulphate, and alkylsulphate radicals. The aliphatic groups can contain, in addition to carbon and hydrogen atoms, ether linkages, and other groups such as amino groups. The longer chain aliphatic groups, e.g., those of 12 carbons, or higher, can be saturated or unsaturated. Specific examples of such quaternary ammonium cationic surfactants of the above general formula are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by other halide (e.g., bromide), acetate, citrate, lactate, glycolate, phosphate nitrate, sulphate, or alkylsulphate.

In a preferred class of cationic surfactant of the above general formula, R¹ is a C₁₆ to C₂₂ saturated or unsaturated, preferably saturated, alkyl chain and R², R³ and R⁴ are each independently selected from CH₃ and CH₂CH₂OH, preferably CH₃.
Specific examples of such preferred quaternary ammonium cationic surfactants for use in forming the conditioning gel phase are cetyltrimethylammonium chloride (CTAC), behenyltrimethylammonium chloride (BTAC) and mixtures thereof.

Mixtures of any of the above-described cationic surfactants may also be suitable.

The level of cationic surfactant suitably ranges from 0.1 to 10%, preferably from 0.2 to 5% and more preferably from 0.25 to 4% (by weight based on the total weight of the composition).

By "high melting point" in the context of this invention is generally meant a melting point of 25°C or higher. Generally, the melting point ranges from 25°C up to 90°C, preferably from 40°C up to 70° C and more preferably from 50°C up to about 65°C.

The high melting point fatty alcohol can be used as a single compound or as a blend or mixture of at least two high melting point fatty alcohols. When a blend or mixture of fatty alcohols is used, the melting point means the melting point of the blend or mixture.

Suitable fatty alcohols of this type have the general formula R-OH, where R is an aliphatic carbon chain. Preferably R is a saturated aliphatic carbon chain comprising from 8 to 30 carbon atoms, more preferably from 14 to 30 carbon atoms and most preferably from 16 to 22 carbon atoms.

R can contain, in addition to carbon and hydrogen atoms, ether linkages, and other groups such as amino groups.

Most preferably, the fatty alcohol has the general formula CH₃(CH₂)ₙ OH, where n is an integer from 7 to 29, preferably from 15 to 21.

Specific examples of suitable fatty alcohols are cetyl alcohol, stearyl alcohol, behenyl alcohol, and mixtures thereof. Cetyl alcohol, stearyl alcohol and mixtures thereof are particularly preferred.

Mixtures of any of the above-described fatty alcohols may also be suitable.

The level of fatty alcohol suitably ranges from 0.01 to 10%, preferably from 0.1 to 8%, more preferably from 0.2 to 7% and most preferably from 0.3 to 6% (by weight based on the total weight of the composition).

The weight ratio of cationic surfactant to fatty alcohol is suitably from 1:1 to 1:10, preferably from 1:1.5 to 1:8, optimally from 1:2 to 1:5.

An aqueous treatment composition for use in step (ii) of the method of the invention may also incorporate other optional ingredients to enhance performance and/or consumer acceptability. Suitable optional ingredients include: preservatives, colouring agents, chelating agents, antioxidants, fragrances, antimicrobials, antidandruff agents, cationic conditioning polymers, styling ingredients, sunscreens, proteins and hydrolysed proteins.

In step (i) of the method of the invention, the hair may be washed with water alone or with shampoo.

In step (ii) of the method of the invention, the washed hair is soaked in the aqueous treatment composition. Generally, any application amount of aqueous treatment composition that covers the hair to be treated suffices. Lesser amounts may be used, for example, if only a section of hair or just the hair tips are to be treated. The aqueous treatment composition is preferably uniformly delivered, for example by working it from the root end to the tip end of the hair.

Preferably, the hair is soaked in the aqueous treatment composition at a temperature from 15 to 40°C, and more preferably at a temperature from 20 to 30°C.

Preferably, the hair is soaked in the aqueous treatment composition for a period ranging from 1 to 60 minutes, more preferably from 3 to 45 minutes.

At the end of the soaking period, it is preferred that the hair is dried or allowed to dry without rinsing the aqueous treatment composition from the hair. The soaked hair may be dried naturally by exposure to air, by use of a heated hair drying appliance, by rubbing with a water-absorbent article, or by a combination of any of these methods.

The aqueous treatment composition may thus remain in contact with the hair after initial application for a period of at least 1 minute, and preferably up until the next wash, e.g. 24 to 72 hours after initial application.

The method of this invention is applied to oxidatively-treated hair.

As used herein, the term "oxidatively-treated hair" means hair which has been subjected to any treatment comprising at least one step of contacting the hair with at least one oxidizing composition. Examples of oxidative treatments for human hair are bleaching, dyeing or perming.

As used herein, the term "oxidizing composition" means a composition comprising at least one oxidizing agent suitable for use on hair, such as hydrogen peroxide, potassium, sodium or ammonium salts of perborate, percarbonate, persulfate and percarbamide, and mixtures thereof. Examples of such compositions are oxidative dye compositions and bleaching compositions.

The invention is further illustrated with reference to the following, non-limiting Examples.

### EXAMPLES

In the Examples, all ingredients are expressed by weight percent of the total formulation, and as level of active ingredient. Comparative Examples (not according to the invention) are indicated by a letter; Examples according to the invention are indicated by a number.

Virgin dark brown European hair switches of length 25 cm and weight 2gms, were treated as follows:
Control: After initial washing, soaked for 30 minutes in water
Example 1: After initial washing, soaked for 30 minutes in a 2% aqueous solution of N-α-acetyl-L-lysine
Example A: After initial washing, soaked for 30 minutes in a 2% aqueous solution of L-lysine
Example B: After initial washing, soaked for 30 minutes in a 2% aqueous solution of N-α-acetyl-L-arginine

At the end of the soaking period the switches were left to dry and clippings from a few fibres were used to measure denaturation temperature Td using DSC. The results are shown in Table 1.

**Table 1**

| **Treatment** | **Average Td (n=3)** |
|---|---|
| Control (water) | 148.8 |
| Example 1 | 153.4 |
| Example A | 148.1 |
| Example B | 148.2 |

It can be seen from the results that treatment of switches according to the method of the invention (Example 1) provides a substantial and significant increase in Td of the treated fibres. By contrast, the comparative treatments (Examples A and B respectively) actually decrease Td.

This shows that the method of the invention can strengthen hair fibres.

Furthermore the method of the invention has the potential to repair hair fibres which have been damaged, for example by oxidative treatments such as bleaching.

The enhanced fibre strengthening provided by the method of the invention may also help to prevent or reduce further fibre damage in the future, whether caused by oxidative treatment or otherwise.

### Example 2

The following formulation illustrates an aqueous treatment composition for use in the method of the invention.

| **Ingredient** | **% activity** | **%w/w raw material** |
|---|---|---|
| Behentrimonium chloride | 70 | 1.1429 |
| Cetearyl alcohol | 100 | 3.0 |
| Perfume | 100 | 0.60 |
| Preservative | 100 | 0.2 |
| Dimethicone emulsion | 70 | 1.429 |
| N-α-acetyl-L-lysine | 100 | 2.0 |
| Water, minors | 100 | To 100% |

## Claims

1. A method of strengthening the fibres of oxidatively treated hair, the method comprising the sequential steps of:
(i) washing the hair;
(ii) soaking the washed hair in an aqueous treatment composition, and
(iii) drying the soaked hair;
**characterised in that** the aqueous treatment composition comprises at least 1% N-acetyl lysine by weight based on the total weight of the composition.

2. A method according to claim 1, in which the isomeric form of the N-acetyl lysine is N-α-acetyl-L-lysine.

3. A method according to claim 1 or claim 2, in which the N-acetyl lysine is used at a level ranging from 1.5 to 2.5% by weight based on the total weight of the composition.

4. A method according to any one of claims 1 to 3, in which the hair is soaked in the aqueous treatment composition at a temperature from 15 to 40°C.

5. A method according to any one of claims 1 to 4, in which the hair is soaked in the aqueous treatment composition for a period ranging from 3 to 45 minutes.

6. A method according to any one of claims 1 to 5, in which, at the end of the soaking period, the hair is dried or allowed to dry without rinsing the aqueous treatment composition from the hair.

7. The use of an aqueous treatment composition comprising at least 1% N-acetyl lysine by weight based on the total weight of the composition, for the strengthening of oxidatively treated hair fibres.

## Patentansprüche

1. Verfahren zur Stärkung der Fasern von oxidativ behandeltem Haar, wobei das Verfahren die folgenden aufeinanderfolgenden Schritte umfasst:
(i) Waschen des Haares;
(ii) Einweichen des gewaschenen Haares in einer wässrigen Behandlungszusammensetzung und
(iii) Trocknen des eingeweichten Haares;
**dadurch gekennzeichnet, dass** die wässrige Behandlungszusammensetzung mindestens 1 Gewichts-% N-Acetyllysin, bezogen auf das Gesamtgewicht der Zusammensetzung, umfasst.

2. Verfahren nach Anspruch 1, bei dem die isomere Form von N-Acetyllysin N-α-Acetyl-L-lysin ist.

3. Verfahren nach Anspruch 1 oder Anspruch 2, bei dem das N-Acetyllysin in einer Menge im Bereich von 1,5 bis 2,5 Gewichts-%, bezogen auf das Gesamtgewicht der Zusammensetzung, verwendet wird.

4. Verfahren nach irgendeinem der Ansprüche 1 bis 3, bei dem das Haar in der wässrigen Behandlungszusammensetzung bei einer Temperatur von 15 bis 40 °C eingeweicht wird.

5. Verfahren nach irgendeinem der Ansprüche 1 bis 4, bei dem das Haar für einen Zeitraum im Bereich von 3 bis 45 Minuten in der wässrigen Behandlungszusammensetzung eingeweicht wird.

6. Verfahren nach irgendeinem der Ansprüche 1 bis 5, bei dem am Ende der Einweichzeit das Haar getrocknet wird oder man es trocknen lässt, ohne die wässrige Behandlungszusammensetzung aus dem Haar auszuspülen.

7. Verwendung einer wässrigen Behandlungszusammensetzung, die mindestens 1 Gewichts-% N-Acetyllysin, bezogen auf das Gesamtgewicht der Zusammensetzung, umfasst, zur Stärkung von oxidativ behandelten Haarfasern.

## Revendications

1. Procédé de renforcement des fibres de cheveux traités par oxydation, le procédé comprenant les étapes successives de :
(i) lavage des cheveux ;
(ii) trempage des cheveux lavés dans une composition de traitement aqueuse, et
(iii) séchage des cheveux trempés ;
**caractérisé en ce que** la composition de traitement aqueuse comprend au moins 1 % de N-acétyl lysine en masse rapporté à la masse totale de la composition.

2. Procédé selon la revendication 1, dans lequel la forme isomère de la N-acétyl lysine est la N-α-acétyl-L-lysine.

3. Procédé selon la revendication 1 ou revendication 2, dans lequel la N-acétyl lysine est utilisée à une teneur de 1,5 à 2,5 % en masse rapportée à la masse totale de la composition.

4. Procédé selon l'une quelconque des revendications 1 à 3, dans lequel les cheveux sont trempés dans la composition de traitement aqueuse à une température de 15 à 40°C.

5. Procédé selon l'une quelconque des revendications 1 à 4, dans lequel les cheveux sont trempés dans la composition de traitement aqueuse sur une durée de 3 à 45 minutes.

6. Procédé selon l'une quelconque des revendications 1 à 5, dans lequel, à la fin de la période de trempage, les cheveux sont séchés ou laissés à sécher sans rinçage de la composition de traitement aqueuse des cheveux.

7. Utilisation d'une composition de traitement aqueuse comprenant au moins 1 % de N-acétyl lysine en masse rapportée à la masse totale de la composition, pour le renforcement de fibres de cheveux traités par oxydation.
